# EUROPÄISCHE PATENTSCHRIFT

(11) **EP 0 374 590 B1**
(45) Veröffentlichungstag und Bekanntmachung des Hinweises auf die Patenterteilung: **15.06.1994**
(21) Anmeldenummer: 89122503.9
(22) Anmeldetag: 06.12.1989
(51) Int. Cl.: A61L 2/08, A61L 27/00, A61F 2/16

(54) **Verfahren zur Sterilisation von intraocularen Linsen**
Method for sterilizing intraocular lenses
Procédé de stérilisation de lentilles intraoculaires

(30) Priorität: 21.12.1988 DE 3843001
(43) Veröffentlichungstag der Anmeldung: 27.06.1990
(73) Patentinhaber: Morcher GmbH, D-70374 Stuttgart (DE)
(72) Erfinder: Morcher, Kurt A., D-7000 Stuttgart 50 (DE)
(74) Vertreter: Manitz, Gerhart, Dr. Dipl.-Phys.

(56) Entgegenhaltungen:
- EP-A- 0 108 661
- EP-A- 0 218 003

## Beschreibung

Die Erfindung betrifft ein Verfahren zur Sterilisation von intraocularen Linsen aus Kunststoffmaterial, wie insbesondere Polymethylmethacrylat (PMMA).

Um beim Implantieren von intraocularen Linsen im menschlichen Auge Infektionen und/oder Irritationen zu vermeiden, muß ein extrem hoher Grad der Sauberkeit und Sterilität der intraocularen Linsen gewährleistet sein.

Grundsätzlich ist es bekannt, die intraocularen zur Sterilisation mit Gasen oder Dämpfen zu behandeln. Derartige Verfahren sind jedoch recht aufwendig, wenn gut reproduzierbare Ergebnisse erreicht werden sollen. Bei der Sterilisation mit Dampf bzw. Gasen muß nämlich eine Vielzahl von Parametern überwacht bzw. berücksichtigt werden, beispielsweise die Wärmeleitfähigkeit der intraocularen Linsen, das Vakuum, die Temperatur und/oder die Luftfeuchtigkeit in der Begasungs- bzw. Bedampfungskammer sowie eine nachfolgende Auslüftzeit. Eine besondere Schwierigkeit liegt darin, daß die Gase bzw. Dämpfe während des Sterilisationsvorganges teilweise vom Kunststoffmaterial der intraocularen Linse aufgenommen werden und dementsprechend immer mit gewissen Rückständen zu rechnen ist.

Es wurde auch bereits versucht, intraoculare Linsen, die in Kochsalzlösung schwimmend in flüssigkeitsdichten Behältnissen verpackt waren, durch Bestrahlung mit Gamma-Strahlen zu sterilisieren. Dabei hat sich jedoch als nachteilig herausgestellt, daß die Gamma-Bestrahlung zur Kristallbildung an der Linsenoberfläche führte.

Aufgabe der Erfindung ist es nun, ein Verfahren zur Sterilisation von intraocularen Linsen so auszugestalten, daß ein besonders hoher Grad der Sterilität und möglichst fremdkörperfreie Linsenoberflächen erreicht werden.

Diese Aufgabe wird erfindungsgemäß dadurch gelöst, daß die intraocularen Linsen in trockenem Zustand in für Bakterien undurchlässige Behältnisse bzw. Beutel verpackt und sodann im verpackten Zustand mit Gamma-Strahlung behandelt werden, um die intraocularen Linsen bei gleichzeitiger Polymerisation des Kunststoffmaterials zu sterilisieren.

Überraschenderweise hat sich herausgestellt, daß die Gamma-Bestrahlung der trockenen intraocularen Linsen zu keinerlei Rückständen an der Linsenoberfläche führt. Damit kann der mit der Gamma-Bestrahlung verbundene Vorteil einer unübertroffenen Sterilisationssicherheit genutzt werden. Es lassen sich ohne weiteres Sicherheitsfaktoren in der Größenordnung von 10⁸ erreichen, d.h. unter 100 Millionen sterilen intraocularen Linsen befindet sich theoretisch nur eine unsterile.

Die Gamma-Bestrahlung bringt den weiteren Vorteil mit sich, daß das Material der intraocularen Linsen zusätzlich polymerisiert wird, d.h. niedermolekulare Bestandteile, beispielsweise Monomere, werden zu hochmolekularen Verbindungen verkettet. Dies ist deshalb von Vorteil, weil die niedermolekularen Materialbestandteile zu sterilen Irritationen des Auges führen können.

Vorzugsweise erfolgt die Gamma-Bestrahlung mit einer Dosis von 28 000 Gy ± 10 %. Diese Dosis ist insofern optimal, als einerseits ein Höchstmaß an Sterilität erreicht wird; andererseits werden bei dieser Dosis bleibende Verfärbungen der intraocularen Linsen vermieden.

Vorübergehende Verfärbungen beruhen auf der Bildung von Radikalen bei der Gamma-Bestrahlung.

Um die Radikale möglichst schnell zu entfernen, ist es zweckmäßig, wenn die intraocularen Linsen in für Gase durchlässigen Behältnissen bzw. Beuteln verpackt werden.

Zusätzlich oder alternativ kann vorgesehen sein, Behältnisse oder Beutel aus solchen Materialien zu verwenden, die die entstehenden Radikale schnell zu binden vermögen.

Ein geeignetes Material für die Behältnisse bzw. Beutel ist beispielsweise Polyäthylen.

Gemäß einer besonders bevorzugten Ausführungsform der Erfindung ist vorgesehen, die intraocularen Linsen nach der Gamma-Bestrahlung im verpackten Zustand zu tempern. Dadurch wird einerseits die Materialstruktur der Linsen noch weiter verbessert. Andererseits werden die bei der Gamma-Bestrahlung gebildeten Radikale beschleunigt ausgetrieben.

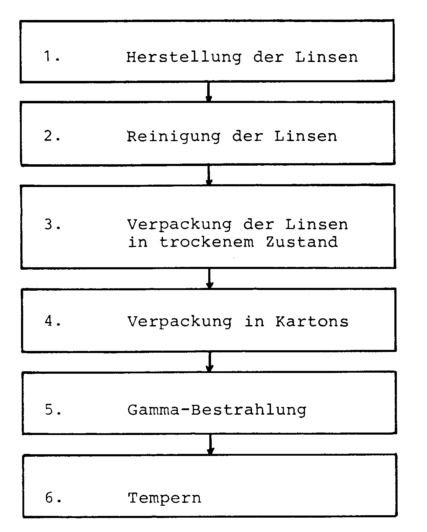

Im übrigen wird hinsichtlich bevorzugter Merkmale der Erfindung auf die Ansprüche sowie die nachfolgende Erläuterung eines besonders bevorzugten Verfahrens anhand der Zeichnung verwiesen.

Dabei zeigt die einzige Figur in schematisierter Weise aufeinanderfolgende Verfahrensabschnitte.

Zunächst werden die intraocularen Linsen in herkömmlicher Weise aus Kunststoffmaterial, insbesondere Polymethylmethacrylat (PMMA), hergestellt bzw. geformt. Dies wird durch Position 1 wiedergegeben.

Danach erfolgt gemäß Position 2 eine Spülung der intraocularen Linsen, um Staub und elektrostatische Aufladungen zu entfernen.

Sodann werden die Linsen bei Position 3 in trockenem Zustand in gasdurchlässigen, jedoch für Bakterien und sonstige Krankheitskeime undurchlässigen Beuteln, z.B. solchen aus Polyäthylen, verpackt.

Danach werden die Beutel gemäß Position 4 in Kartons od.dgl. eingelegt, die nachfolgend palettiert werden können.

Die gegebenenfalls palettierten Kartons werden gemäß Position 5 durch eine Bestrahlungskammer geleitet, in der sie, und damit die intraocularen Linsen, einer Gamma-Strahlung ausgesetzt werden. Zur Erzeugung der Gamma-Strahlung dient in diesem Falle eine Kobalt-60-Quelle. Abschließend werden die Kartons bzw. die verpackten und bereits sterilisierten intraocularen Linsen in einer Wärmekammer getempert. Dabei werden die intraocularen Linsen einer Temperatur von 60°C ausgesetzt, um die bei der Gamma-Bestrahlung gebildeten Radikale und die dadurch verursachten vorübergehenden Verfärbungen der Linsen auszutreiben bzw. rückgängig zu machen.

Bei der Gamma-Bestrahlung wird neben den Linsen auch das Verpackungsmaterial sterilisiert. Durch Verpackung der Linsen in Doppelbeutel od.dgl. kann also gewährleistet werden, daß die Linsen nach Öffnen des Außenbehältnisses in einen außenseitig sterilen Innenbehältnis vorliegen.

Im übrigen wird durch die Gamma-Bestrahlung eine Polymerisation niedermolekularer Materialbestandteile der intraocularen Linsen bewirkt. Dabei sinkt der Anteil der Monomere und Oligomere auf etwa 1/10 des vor der Bestrahlung liegenden Anteiles ab. Dies ist im Hinblick auf eine gute Verträglichkeit der intraocularen Linse im menschlichen Auge von großem Vorteil.

## Patentansprüche

1. Verfahren zur Sterilisation von intraocularen Linsen aus Kunststoffmaterial, wie insbesondere Polymethylmethacrylat (PMMA),
dadurch gekennzeichnet,
daß die intraocularen Linsen in trockenem Zustand in für Bakterien und Krankheitskeime undurchlässigen Behältnissen bzw. Beuteln verpackt und sodann im verpackten Zustand mit Gamma-Strahlung behandelt werden, um die Linsen bei gleichzeitiger Polymerisation des Kunststoffmateriales zu sterilisieren.

2. Verfahren nach Anspruch 1, dadurch gekennzeichnet, daß die intraocularen Linsen mit einer Dosis von 28 000 Gy ± 10 % bestrahlt werden.

3. Verfahren nach einem der Ansprüche 1 oder 2, dadurch gekennzeichnet, daß die intraocularen Linsen in für Gas durchlässige Behältnisse bzw. Beutel verpackt werden.

4. Verfahren nach einem der Ansprüche 1 bis 3, dadurch gekennzeichnet, daß die intraocularen Linsen in Behältnissen bzw. Beuteln verpackt werden, deren Material bei der Gamma-Bestrahlung der intraocularen Linsen entstehende Radikale zu binden vermag.

5. Verfahren nach einem der Ansprüche 1 bis 4, dadurch gekennzeichnet, daß die intraocularen Linsen in Behältnissen aus Polyäthylen verpackt werden.

6. Verfahren nach einem der Ansprüche 1 bis 5, dadurch gekennzeichnet, daß die intraocularen Linsen vor ihrer Verwendung in den Behältnissen bzw. Beuteln so lange verbleiben, bis bei der Gamma-Bestrahlung auftretende Verfärbungen bzw. Radikale zurückgebildet bzw. entwichen sind.

7. Verfahren nach einem der Ansprüche 1 bis 6, dadurch gekennzeichnet, daß die intraocularen Linsen nach der Gamma-Bestrahlung im verpackten Zustand getempert werden.

8. Verfahren nach Anspruch 7, dadurch gekennzeichnet, daß die Temperung in einer Unterdruckkammer erfolgt.

## Claims

1. Method for the sterilization of intraocular lenses of plastic material, such as in particular polymethylmethacrylate (PMMA), characterised in that the intraocular lenses are packed in the dry state in containers or bags which are impermeable for bacteria and germs causing illness, and are then treated in the packed state with gamma radiation in order to sterilise the lenses with simultaneous polymerization of the plastic material.

2. Method in accordance with claim 1,
characterised in that the intraocular lenses are irradiated with a dose of 28,000 Gy ± 10 %.

3. Method in accordance with one of the claims 1 or 2, characterised in that the intraocular lenses are packed in containers or bags which are permeable to gas.

4. Method in accordance with one of the claims 1 to 3, characterised in that the intraocular lenses are packed in containers or bags, the material of which is able to bind radicals arising during the gamma irradiation of the intraocular lenses.

5. Method in accordance with one of the claims 1 to 5, characterised in that the intraocular lenses are packed in containers of polyethylene.

6. Method in accordance with one of the claims 1 to 5, characterised in that the intraocular lenses remain, prior to their use, in the containers or bags for a sufficient length of time until discolorations or radicals which have arisen with the gamma irradiation have reformed or escaped.

7. Method in accordance with one of the claims 1 to 6, characterised in that the intraocular lenses are tempered in the packed state after the gamma irradiation.

8. Method in accordance with claim 7,
characterised in that the tempering takes place in a vacuum chamber.

## Revendications

1. Procédé pour la stérilisation de lentilles intra-oculaires en résines synthétiques, en particulier en polyméthacrylate de méthyle (PMMA), caractérisé en ce que l'on emballe les lentilles intra-oculaires à l'état sec dans des récipients ou sachets imperméables aux bactéries et aux germes de maladies puis on les traite, à l'état emballé, par des rayons gamma, pour stériliser les lentilles par polymérisation de la résine synthétique.

2. Procédé selon la revendication 1, caractérisé en ce que l'on irradie les lentilles intra-oculaires à une dose de 28 000 Gy + 10%.

3. Procédé selon la revendication 1 ou 2, caractérisé en ce que les lentilles intra-oculaires sont emballées dans des récipients ou sachets perméables aux gaz.

4. Procédé selon une des revendications 1 à 3, caractérisé en ce que les lentilles intra-oculaires sont emballées dans des récipients ou sachets dont le matériau est capable de fixer les radicaux libres formés à l'exposition des lentilles intra-oculaires aux rayons gamma.

5. Procédé selon une des revendications 1 à 4, caractérisé en ce que les lentilles intra-oculaires sont emballées en récipients de polyéthylène.

6. Procédé selon une des revendications 1 à 5, caractérisé en ce que, avant leur utilisation, les lentilles intra-oculaires sont maintenues dans les récipients ou sachets jusqu'à élimination ou recombinaison des radicaux libres formés à l'exposition aux rayons gamma et disparition des colorations provoquées par ces radicaux libres.

7. Procédé selon une des revendications 1 à 6, caractérisé en ce que, après l'exposition aux rayons gamma, les lentilles intra-oculaires sont soumises à chauffage à l'état emballé.

8. Procédé selon la revendication 7, caractérisé en ce que le chauffage est réalisé dans une chambre sous vide.
